# EUROPEAN PATENT APPLICATION

(11) **EP 4 505 949 A1**
(43) Date of publication of application: **12.02.2025**
(21) Application number: 23799286.2
(22) Date of filing: 05.05.2023
(51) Int. Cl.: A61B 17/12

(54) **HOOK AND OCCLUSION DEVICE**

(30) Priority: 05.05.2022 CN 202210481641
(71) Applicant: Shanghai Shape Memory Alloy Co., Ltd., Shanghai, 201612 (CN)
(72) Inventor: CHEN, Juan, Shanghai 201612 (CN); ZHANG, Xiang, Shanghai 201612 (CN); WANG, Yunbing, Shanghai 201612 (CN); HU, Jinpeng, Shanghai 201612 (CN); WANG, Fan, Shanghai 201612 (CN); LIU, Can, Shanghai 201612 (CN); LIU, Dezhong, Shanghai 201612 (CN); PAN, Xiangbin, Shanghai 201612 (CN)
(74) Representative: Canzler & Bergmeier Patentanwälte Partnerschaft mbB
(86) International application number: PCT/CN2023/092273
(87) International publication number: WO 2023/213300

(57) **Abstract**

Disclosed is a hook and an occlusion device. The hook is used for being fixed to the occlusion device. The occlusion device is formed by interweaving a first-direction line and a second-direction line. The hook comprises a positioning body and a hook part, wherein the positioning body is provided with a first positioning part and a second positioning part, and the first positioning part and the second positioning part are used for limiting the hook to the first-direction line and the second-direction line, respectively, or the first positioning part and the second positioning part are used for simultaneously limiting, in different directions of force, the hook in the axial direction to the first-direction line/the second-direction line; the hook part is formed by bending the positioning body, an included angle α is formed between the projection of the hook part on the positioning body and the axis of the positioning body, and the value range of the included angle α is greater than 0 degrees and smaller than 90 degrees. The hook provided by the present invention can be fixed to the occlusion device without other auxiliary parts such as suture lines, is excellent in stability and firmness, and can increase the supporting strength of the occlusion device.

## Description

### FIELD

The present invention relates to the technical field of medical devices, and particularly to a hook and an occlusion device.

### BACKGROUND

When patients experience atrial fibrillation, thrombi may be formed in the left atrial appendage, and thromboembolism poses risks of long-term disability or death. Left atrial appendage occlusion may reduce these risks by occluding the left atrial appendage. Currently, commonly used left atrial appendage occluders often have issues with inadequate endothelialization on the surfaces, and the surfaces of the occluders are prone to thrombus formation, which becomes one of the complications that seriously threaten the health and life of patients. Additionally, most existing left atrial appendage occluders are made of nickel-titanium alloy, and due to the non-degradable characteristics, the stents remain in the human body for a long time after implantation, leading to a series of chronic problems such as occluder detachment, inadequate endothelialization of the occluders, device-related thrombi, and wear of the device on the surrounding tissues of the atrial appendage.

The emergence of degradable left atrial appendage occluders may significantly alleviate the above-mentioned problems. However, degradable materials have poor support and memory properties, making them prone to the risk of detachment after implantation. In order to prevent detachment, in the related art, memory hooks with better support properties will be added to the degradable left atrial appendage occluders and combined with the degradable left atrial appendage occluders by sutures. However, this significantly increases the volume of non-degradable materials and also raises the risk of detachment of the memory hooks. How to effectively combine a degradable material with a smaller volume of a non-degradable material in an occluder to balance the above-mentioned problems is a problem that needs to be solved urgently in the art at present.

### SUMMARY

In order to overcome the problems existing in the related art to at least some extent, one of the objects of the present application is to provide a hook, which can be mounted on an occluder through its own structure without other auxiliary parts, such as sutures for fixation, and is excellent in stability and firmness, thereby increasing the supporting strength of the occluder.

Another object of the present application is to provide an occluder, and the occluder is provided with the above-mentioned hooks. The occluder has a stable occluding effect and a high supporting strength of an occluding disc. The specific technical solutions are as follows.

According to a first aspect of embodiments of the present application, a hook is provided and used for being fixed to an occluder, where the occluder is formed by interweaving a first-direction wire and a second-direction wire, and the hook includes a positioning body and hook portions;
the positioning body is provided with a first positioning portion and a second positioning portion; the first positioning portion is configured to limit the hook to one of the first-direction wire and the second-direction wire, and the second positioning portion is configured to limit the hook to the other of the first-direction wire and the second-direction wire;
or the first positioning portion and the second positioning portion are configured to limit, in different directions of force, the hook in an axial direction to the first-direction wire or the second-direction wire;
the hook portion is formed by bending the positioning body; an included angle α is formed between a projection of the hook portion on the positioning body and an axis of the positioning body, and the included angle α is in a range of greater than 0 degrees and less than 90 degrees.

In some implementations, the first positioning portion includes at least two first positioning holes arranged on the positioning body, and the two first positioning holes are distributed along the axial direction; the second positioning portion includes at least two second positioning holes; one of the first-direction wire and the second-direction wire is threaded through the first positioning holes, and the other of the first-direction wire and the second-direction wire is threaded through the second positioning holes.

In some implementations, a connecting line between the second positioning holes and a connecting line between the first positioning holes have an included angle β, and a value of the included angle β is equal to a value of an included angle between the first-direction wire and the second-direction wire.

In some implementations, there are two first positioning holes, and the two first positioning holes are arranged at two ends of the positioning body, respectively.

In some implementations, bulging portions are formed on the positioning body corresponding to the first positioning holes and the second positioning holes.

In some implementations, one hook portion is provided beside each first positioning hole; the hook portions are formed by extending and bending the positioning body, and bending angles γ formed between the hook portions and the positioning body are the same.

In some implementations, the positioning body has a first edge and a second edge which are opposite to each other; the first positioning portion includes first parts and second parts which are provided on the first edge and the second edge, respectively, and the first parts and the second parts are detachably connected; the first edge and the second edge are bendable towards each other to fix the positioning body to the first-direction wire or the second-direction wire by buckling the first parts and the second parts.

In some implementations, the first parts and the second parts are clasps buckled with each other.

In some implementations, the first parts are several catching grooves arranged on the first edge in the axial direction, and the second parts are connecting convex portions which correspond to and are adapted to the catching grooves one by one.

In some implementations, the second positioning portion includes at least one pair of oppositely provided barbs, and the two barbs are provided along the axial direction of the positioning body.

In some implementations, the second positioning portion includes two pairs of barbs, and the two pairs of barbs are provided flush in the axial direction.

In some implementations, the hook portion is formed by bending the positioning body after cutting.

In some implementations, the hook portion includes a strip which is cut from an edge of the positioning body and connected to the positioning body at the root; the strip is bent away from a bending direction of the barb.

In some implementations, the projection of the hook portion on the positioning body is located on a first side or a second side of the axis of the positioning body, where the first side is an opposite side of the second side.

In some implementations, when the projection of the hook portion on the positioning body is located on the first side of the axis of the positioning body, the first positioning portion is configured to limit the hook in the axial direction to the first-direction wire; when the projection of the hook portion on the positioning body is located on the second side of the axis of the positioning body, the first positioning portion is configured to limit the hook in the axial direction to the second-direction wire.

An occluder is provided and formed by interweaving a first-direction wire and a second-direction wire, including:
a fixing disc, provided with several hooks according to any one of the above-mentioned implementations and used for being fixed in a left atrial appendage through the hooks; and
an occluding disc having a diameter greater than the fixing disc, configured to occlude the left atrial appendage.
In some implementations, the hooks are provided on the first-direction wire and the second-direction wire of the fixing disc, and hanging directions of the hooks are the same.

In some implementations, the hooks include first hooks and second hooks; the first hook is limited to the first-direction wire through the first positioning portion, and the second hook is limited to the second-direction wire through the first positioning portion, where the hook portions of the first hook and the hook portions of the second hook are oriented in the same direction.

In some implementations, the positioning body has a first edge and a second edge which are opposite to each other; the first positioning portion includes first parts and second parts which are provided on the first edge and the second edge, respectively, and the first parts and the second parts are detachably connected;
the second positioning portion includes at least one pair of oppositely provided barbs, and the two barbs are provided along the axial direction of the positioning body;
the positioning body wraps around the first-direction wire or the second-direction wire after bending the first edge and the second edge towards each other, the first parts and the second parts of the positioning body are buckled with each other so that the positioning body sheathes the first-direction wire or the second-direction wire, and the barbs puncture the first-direction wire or the second-direction wire for fixing under an external pressure;
the first-direction wire and the second-direction wire intersect to form a node, and the two barbs are fixed at two sides of the node on the first-direction wire or the second-direction wire, respectively.

In some implementations, the hooks include first hooks and second hooks; the positioning body of the first hook wraps around the first-direction wire after bending the first edge and the second edge towards each other, and the positioning body of the second hook wraps around the second-direction wire after bending the first edge and the second edge towards each other, where the hook portion of the first hook and the hook portion of the second hook are oriented in the same direction.

In some implementations, when the first hook is mounted on the first-direction wire of the fixing disc and the second hook is mounted on the second-direction wire of the fixing disc, an opening between the hook portion and the positioning body of the first hook faces the occluding disc, and an opening between the hook portion and the positioning body of the second hook faces the occluder.

In some implementations, the hooks are arranged in a wavy line along a circumferential direction of the fixing disc.

The technical solutions provided by the embodiments of the present application have at least the following beneficial effects.

The hook of the present application, based on the positioning body, may be directly fixed to the occluder without other auxiliary structures such as sutures, thereby reducing the proportion of non-degradable materials in the occluder. Specifically, the positioning body includes the first positioning portion and the second positioning portion. The positioning body may be fixed to the first-direction wire through the first positioning portion and fixed to the second-direction wire through the second positioning portion; or the positioning body may be fixed to the second-direction wire through the first positioning portion and fixed to the first-direction wire through the second positioning portion. The occluder is formed by interweaving the first-direction wire and the second-direction wire so that the hook may be stably fixed to the occluder through the first positioning portion and the second positioning portion. In addition, the first positioning portion and the second positioning portion may be fixed to the same direction wire at the same time under the premise of different directions of force, for example, being fixed to the first-direction wire at the same time or being fixed to the second-direction wire at the same time, thereby stably fixing the hook to the occluder under the complementary condition.

According to the present application, the hook portion is formed by bending the positioning body. The hook is fixed in the axial direction to the first-direction wire/the second-direction wire. Thus, the positioning body is fixed to the occluding disc with the axial direction parallel to the first-direction wire or the second-direction wire. When the included angle α between the projection of the hook portion on the positioning body and the axis of the positioning body is in a range of greater than 0 degrees and less than 90 degrees, a stable fixed angle between the hook portion and the left atrial appendage may be ensured when the hook is fixed to the occluder.

It is to be understood that the foregoing general description and the following detailed description are exemplary and explanatory only and cannot limit the present application.

### BRIEF DESCRIPTION OF THE DRAWINGS

The accompanying drawings, which are incorporated in and constitute a part of this specification, illustrate embodiments consistent with the present application and, together with the description, serve to explain the principles of the present application.
FIG. 1 is a schematic diagram showing how hooks are mounted on an occluder according to embodiment 1 of the present invention;
FIG. 2 is a schematic structural diagram of a hook according to embodiment 1 of the present invention;
FIG. 3 is a side view of the hook of FIG. 2;
FIG. 4 is a schematic structural diagram of another hook according to embodiment 1 of the present invention;
FIG. 5 is a schematic diagram showing how hooks are mounted on an occluder according to embodiment 2 of the present invention;
FIG. 6 is a schematic structural diagram of a hook according to embodiment 2 of the present invention;
FIG. 7 is a top view of the hook of FIG. 6;
FIG. 8 is a partially enlarged schematic diagram of a first positioning portion at A in FIG. 7;
FIG. 9 is an expanded schematic diagram of a hook according to embodiment 2 of the present invention;
FIG. 10 is a schematic diagram showing a connection relationship between a hook and a first-direction wire/a second-direction wire according to embodiment 2 of the present invention;
FIG. 11 is a sectional view taken along an A-A line in FIG. 10;
FIG. 12 is a schematic structural diagram of an occluder according to an embodiment of the present invention;
FIG. 13 is a force analysis diagram showing the arrangement of hooks according to an embodiment of the present invention;
FIG. 14 is a schematic diagram showing a mounting position of an occluder in a left atrial appendage according to an embodiment of the present invention;
FIG. 15 is a schematic diagram of a side profile of an occluder according to an embodiment of the present invention;
FIG. 16 is a schematic diagram of an occluder being released through a delivery sheath according to an embodiment of the present invention; and
FIG. 17 is a schematic diagram of an occluder being retrieved through a delivery sheath according to an embodiment of the present invention.

Description of Reference Numerals:
100-first hook; 110-positioning body; 111-first positioning portion; 1111-first part; 1112-second part; 112-second positioning portion; 1121-barb; 120-hook portion; 130-bulging portion; and 140-opening;
200-second hook;210-second hook portion; and M-axis of positioning body;
300-occluder; 310-fixing disc; 320-occluding disc; 330-first-direction wire; and 340-second-direction wire;
400-left atrial appendage; and
500-delivery sheath.

### DETAILED DESCRIPTION

Exemplary embodiments are described in detail here, and examples are shown in the accompanying drawings. When the following description relates to drawings, the same number in different drawings denotes the same or similar elements, unless otherwise indicated. The implementations described in the following exemplary embodiments do not represent all implementations consistent with the present application. Rather, they are merely examples of methods consistent with some aspects of the present application as detailed in the appended claims.

In the related art, a hook with a memory property is usually provided on a degradable occluder to increase the support strength and prevent the occluder from falling off. However, auxiliary structures such as sutures are usually required to fix the hook and the occluder, which increases the proportion of non-degradable materials. Meanwhile, an additional component increases the risk of the memory hook falling off from the occluder, or due to the design defect of the structure, it is not conducive to the effective combination of the hook and the occluder.

Based on the analysis and discovery of the above-mentioned problems, the present application is proposed.

The present application provides a hook, used for being fixed to an occluder. The occluder is formed by interweaving a first-direction wire and a second-direction wire, and the hook includes a positioning body and hook portions.

The positioning body is provided with a first positioning portion and a second positioning portion. The first positioning portion is configured to limit the hook to the first-direction wire axially, and the second positioning portion is configured to limit the hook to the second-direction wire; or the first positioning portion and the second positioning portion are configured to limit, in different directions of force, the hook to the first-direction wire or the second-direction wire axially.

The hook portion is formed by bending the positioning body. An included angle α is formed between a projection of the hook portion on the positioning body and an axis of the positioning body, and the included angle α is in a range of greater than 0 degrees and less than 90 degrees.

The hook of the present application, based on the positioning body, may be directly fixed to the occluder without other auxiliary structures such as sutures, thereby reducing the proportion of non-degradable materials in the occluder. Specifically, the positioning body includes the first positioning portion and the second positioning portion. The positioning body may be fixed to the first-direction wire through the first positioning portion and fixed to the second-direction wire through the second positioning portion; or the positioning body may be fixed to the second-direction wire through the first positioning portion and fixed to the first-direction wire through the second positioning portion. The occluder is formed by interweaving the first-direction wire and the second-direction wire so that the hook may be stably fixed to the occluder through the first positioning portion and the second positioning portion. In addition, the first positioning portion and the second positioning portion may be fixed to the same direction wire at the same time under the premise of different directions of force, for example, being fixed to the first-direction wire at the same time or being fixed to the second-direction wire at the same time, thereby stably fixing the hook to the occluder under the complementary condition.

According to the present application, the hook portion is formed by bending the positioning body. The hook is fixed in the axial direction to the first-direction wire/the second-direction wire. Thus, the positioning body is fixed to the occluding disc with the axial direction parallel to the first-direction wire or the second-direction wire. When the included angle α between the projection of the hook portion on the positioning body and the axis of the positioning body is in a range of greater than 0 degrees and less than 90 degrees, a stable fixed angle between the hook portion and the left atrial appendage may be ensured when the hook is fixed to the occluder.

In some embodiments, the first positioning portion includes at least two first positioning holes arranged on the positioning body in the axial direction. The second positioning portion includes at least two second positioning holes arranged between the first positioning holes. The first-direction wire and the second-direction wire are threaded through the first positioning holes and the second positioning holes, respectively. Preferably, a connecting line between the second positioning holes and a connecting line between the first positioning holes have an included angle β, and a value of the included angle β is equal to a value of an included angle between the first-direction wire and the second-direction wire. In these embodiments, the first positioning portion and the second positioning portion each include a plurality of positioning holes arranged on the positioning body. The included angle between the connecting line of the first positioning holes and the connecting line of the second positioning holes is the same as an included angle between the first-direction wire and the second-direction wire. Thus, illustratively, the first-direction wire may smoothly penetrate the first positioning hole, and the second-direction wire may smoothly penetrate the second positioning hole. Therefore, based on the intersected arrangement of the first-direction wire and the second-direction wire, the first positioning portion and the second positioning portion may limit the hook to the occluder in different directions, and in the case where the occluder is not damaged, the hook will not disengage from the occluder.

Preferably, there are two first positioning holes, and the two first positioning holes are arranged at two ends of the positioning body, respectively. Preferably, bulging portions are formed on the positioning body corresponding to the first positioning holes and the second positioning holes. In these embodiments, in order to reduce production costs and balance stress stability, two first positioning holes and two second positioning holes are provided. Providing the first positioning holes and the second positioning holes on the bulging portions of the positioning body may increase the stress strength of the positioning body and improve its service life.

In some embodiments, one hook portion is provided beside each first positioning hole; the hook portions are formed by extending and bending the positioning body, and bending angles γ formed between the hook portions and the positioning body are the same. In these embodiments, a plurality of hook portions may be provided, and the positions of the hook portions are not limited to be provided beside the first positioning holes. However, if the hook portions are provided beside the first positioning holes, the first positioning holes may be fixed to the first-direction wire during use. Thus, the stability is good, and a stable connection of the hook portions in the left atrial appendage may be ensured. Furthermore, the bending angles γ formed between the hook portions and the positioning body should be the same, and in this way, when the hook portions are fixed in the left atrial appendage, directions of force of the hook portions can be ensured to be consistent, increasing the fixing strength of the hook portions in the left atrial appendage and preventing slipping.

In some embodiments, the positioning body has a first edge and a second edge which are opposite to each other; the first positioning portion includes first parts and second parts which are provided on the first edge and the second edge, respectively, and the first parts and the second parts are detachably connected; the first edge and the second edge are bendable towards each other to fix the positioning body to the first-direction wire/the second-direction wire by buckling the first parts and the second parts. In these embodiments, based on the buckling of the first parts and the second parts in the first positioning portion, the positioning body has a cylindrical shape sheathing the first-direction wire/second-direction wire during use so as to be limited to the first-direction wire/second-direction wire in the axial direction.

In some embodiments, the first parts and the second parts are clasps buckled with each other.

In some embodiments, the first parts are several catching grooves arranged on the first edge in the axial direction, and the second parts are connecting convex portions which correspond to and are adapted to the catching grooves one by one.

In some embodiments, the second positioning portion includes at least one pair of oppositely provided barbs, and the two barbs are provided along the axial direction of the positioning body. In these embodiments, the second positioning portion radially limits the positioning body on the corresponding first-direction wire/second-direction wire through the oppositely arranged barbs, i.e., limiting the hook in a direction different from the first positioning portion, so that the hook may be stably fixed to the first-direction wire/second-direction wire. Preferably, the second positioning portion includes two pairs of barbs, and the two pairs of barbs are provided flush in the axial direction. In order to further increase the stability of the connection between the hook and the occluder, a plurality of pairs of barbs may be provided, and from the volume of the positioning body and other practical situations, two pairs of flush barbs may ensure the stability of the connection.

In some embodiments, the hook portion is formed by bending the positioning body after cutting. In these embodiments, the hook portion is also formed by bending the positioning body, except that the hook portion in these embodiments is formed by bending the positioning body after cutting. Preferably, the hook portion is a strip which is cut from an end portion of the positioning body in the axial direction and connected to the positioning body at the root. The strip is bent away from a bending direction of the barb.

In some embodiments, the projection of the hook portion on the positioning body is located on a first side or a second side of the axis of the positioning body, where the first side is an opposite side of the second side. In these embodiments,

In some embodiments, when the projection of the hook portion on the positioning body is located on the first side of the axis of the positioning body, the first positioning portion is configured to limit the hook in the axial direction to the first-direction wire; when the projection of the hook portion on the positioning body is located on the second side of the axis of the positioning body, the first positioning portion is configured to limit the hook in the axial direction to the second-direction wire.

According to a second aspect of embodiments of the present application, an occluder is provided. The occluder is formed by interweaving a first-direction wire and a second-direction wire, including:
a fixing disc, provided with several hooks and used for being fixed in a left atrial appendage through the hooks; and
an occluding disc having a diameter greater than the fixing disc, configured to occlude the left atrial appendage.

In these embodiments, the above-mentioned hooks are provided on the fixing disc of the occluder to fix the occluder in the left atrial appendage. Based on the structural characteristics of the hook itself, the hook may be directly fixed to the occluder without other auxiliary structures, is firmly connected to the occluder, and is not easy to fall off, thereby enhancing the occluding effect of the occluder.

In some embodiments, the hooks are provided on the first-direction wire and the second-direction wire of the fixing disc, and hanging directions of the hooks are the same. In these embodiments, whether the positioning body is fixed to the fixing disc in a strip shape or a rolled cylindrical shape, based on different bending directions of the hook portions in the hook, i.e., when the hook is fixed to the first-direction wire in the axial direction, the projection of the hook portion on the positioning body is located on the first side of the axis of the positioning body, and when the hook is fixed to the second-direction wire in the axial direction, the projection of the hook portion on the positioning body is located on the second side of the axis of the positioning body. At this time, the hanging directions of the hooks are the same. Therefore, it can be understood that two mirror-symmetrical hooks are axially fixed to the first-direction wire and the second-direction wire, respectively. The hooks are provided in two directions and may form two forms. During use, the hooks of the two forms are fixed to the first-direction wire and the second-direction wire, respectively, and the hook portions of the two forms have the same hanging direction in the left atrial appendage so that a hanging holding force may be applied in the same direction to prevent the occluder from unhooking. Providing hooks on both the first-direction wire and the second-direction wire is equivalent to providing reinforcing ribs in two directions, thereby greatly enhancing the supporting force of the occluder.

In some embodiments, the hooks include first hooks and second hooks; the first hook is limited to the first-direction wire through the first positioning portion, and the second hook is limited to the second-direction wire through the first positioning portion, where the hook portion of the first hook and the hook portion of the second hook are oriented in the same direction.

In some embodiments, the hooks are arranged in a wavy line along a circumferential direction of the fixing disc. In these embodiments, the fixing disc is formed by weaving the first-direction wire and the second-direction wire so that the first-direction wire and the second-direction wire would form several diamond meshes. The first hook and the second hook are arranged in a wavy line so that they are necessarily located on two adjacent sides of a certain diamond mesh or near adjacent vertices of the diamond mesh, which may increase the supporting strength of the occluder and make the occluding effect more stable.

In some embodiments, the positioning body has a first edge and a second edge which are opposite to each other; the first positioning portion includes first parts and second parts which are provided on the first edge and the second edge, respectively, and the first parts and the second parts are detachably connected.

The second positioning portion includes at least one pair of oppositely provided barbs, and the two barbs are provided along the axial direction of the positioning body.

The positioning body wraps around the designated first-direction wire/the designated second-direction wire after bending the first edge and the second edge towards each other, the first parts and the second parts of the positioning body are buckled with each other so that the positioning body sheathes the designated first-direction wire/the designated second-direction wire, and the barbs puncture the designated first-direction wire/the designated second-direction wire for fixing under an external pressure.

The first-direction wire and the second-direction wire intersect to form a node, and the two barbs are fixed at two sides of the node on the designated first-direction wire/the designated second-direction wire, respectively. In these embodiments, when the two barbs are provided on two sides of the node, the stability of the connection between the hook and the fixing disc may be further improved.

In some embodiments, the hooks include first hooks and second hooks; the positioning body of the first hook wraps around the first-direction wire after bending the first edge and the second edge towards each other, and the positioning body of the second hook wraps around the second-direction wire after bending the first edge and the second edge towards each other, where the hook portion of the first hook and the hook portion of the second hook are oriented in the same direction.

In some embodiments, when the first hook is mounted on the first-direction wire of the fixing disc and the second hook is mounted on the second-direction wire of the fixing disc, an opening between the hook portion and the positioning body of the first hook faces the occluding disc, and an opening between the hook portion and the positioning body of the second hook faces the occluding disc.

In order to understand the hooks and the occluders provided by the embodiments of the present disclosure more clearly, specific embodiments of the present disclosure will now be described with reference to the accompanying drawings, in which the thickness of the wires or the size of the structural elements shown in the drawings, etc. may be shown in an exaggerated form for clarity and convenience of the description.

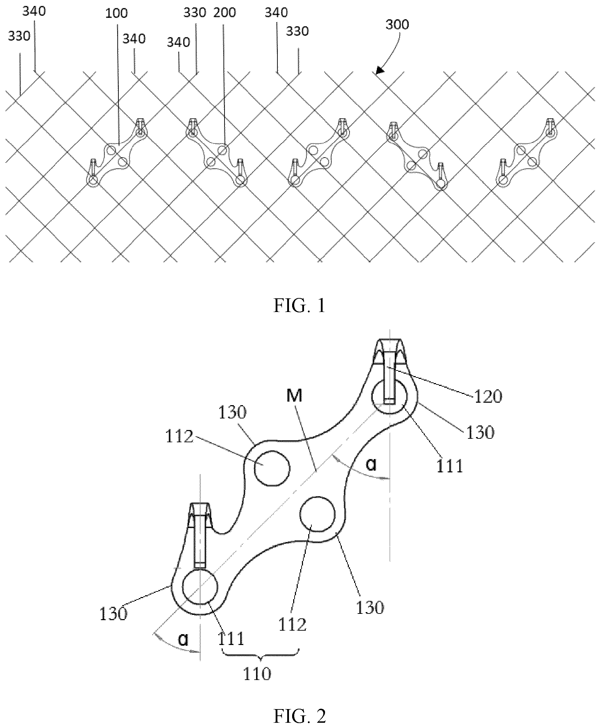

Referring to FIG. 1, this embodiment provides a hook, which is formed by cutting and heat treating a memory metal material and used with an occluder 300 formed by weaving a wire material. The memory metal material may be either a non-degradable memory material or a degradable memory metal material, such as a nickel-titanium wire, a high molecular material wire, or a degradable magnesium alloy. A developing coating is attached on the surface of the hook so that the doctor can clearly see the position of the hook in the left atrial appendage under angiography during operation so as to determine whether the occluder is stable. The hook is mainly fixed to the occluder 300 by threading a wire. The occluder 300 is formed by interweaving a first-direction wire 330 and a second-direction wire 340, including an outer layer of the occluder, a flow blocking membrane, 3: a forming ring, hooks, and a welding ball. The outer layer of the occluder as a framework component includes the fixing disc and the occluding disc formed by weaving and heat treating a degradable wire material. The flow blocking membrane is fixed to the outer layer of the occluder through a degradable wire, thereby producing a flow blocking and isolation effect. The hook is connected and fixed to the fixing disc of the outer layer of the occluder by perforation or press-fitting.

Referring to FIGS. 2-4 and 6-9, the hook has a positioning body 110 and hook portions 120. The positioning body 110 is provided with a first positioning portion 111 and a second positioning portion 112. The first positioning portion 111 and the second positioning portion 112 are configured to limit the hook to the first-direction wire 330 and the second-direction wire 340, respectively, or the first positioning portion 111 and the second positioning portion 112 are configured to simultaneously limit, in different directions of force, the hook in an axial direction to the first-direction wire 330/the second-direction wire 340. The hook portion is formed by bending the positioning body 110. An included angle α is formed between a projection of the hook portion 120 on the positioning body 110 and an axis M of the positioning body 110, and the included angle α is in a range of greater than 0 degrees and less than 90 degrees.

The hook of the present application, based on the positioning body 110, may be directly fixed to the occluder 300 without other auxiliary structures such as sutures, thereby reducing the proportion of non-degradable materials in the occluder 300. Specifically, the positioning body 110 includes the first positioning portion 111 and the second positioning portion 112. The positioning body 110 may be fixed to the first-direction wire 330 through the first positioning portion 111 and fixed to the second-direction wire 340 through the second positioning portion 112; or the positioning body 110 may be fixed to the second-direction wire 340 through the first positioning portion 111 and fixed to the first-direction wire 340 through the second positioning portion 112. The occluder 300 is formed by interweaving the first-direction wire 330 and the second-direction wire 340 so that the hook may be stably fixed to the occluder 300 through the first positioning portion 111 and the second positioning portion 112. In addition, the first positioning portion 111 and the second positioning portion 112 may be fixed to the same direction wire at the same time under the premise of different directions of force, for example, being fixed to the first-direction wire 330 at the same time or being fixed to the second-direction wire 340 at the same time, thereby stably fixing the hook to the occluder 300 under the complementary condition. According to the present application, the hook portion 120 is formed by bending the positioning body 110. The hook is fixed to the first-direction wire 330/the second-direction wire 340 axially. Thus, the positioning body 110 is fixed to the occluding disc 300 with the axial direction parallel to the first-direction wire 330 or the second-direction wire 340. When the included angle α between the projection of the hook portion 120 on the positioning body 110 and the axis M of the positioning body 110 is in a range of greater than 0 degrees and less than 90 degrees, a stable fixed angle between the hook portion 120 and the left atrial appendage 400 may be ensured when the hook is fixed to the occluder 300.

The hook of the present application, based on the positioning body 110, may be directly fixed to the occluder 300 without other auxiliary structures such as sutures, thereby reducing the proportion of non-degradable materials in the occluder 300. Specifically, the positioning body 110 includes the first positioning portion 111 and the second positioning portion 112. The positioning body 110 may be fixed to the first-direction wire 330 through the first positioning portion 111 and fixed to the second-direction wire 340 through the second positioning portion 112; or the positioning body 110 may be fixed to the second-direction wire 340 through the first positioning portion 111 and fixed to the first-direction wire 330 through the second positioning portion 112. The occluder 300 is formed by interweaving the first-direction wire 330 and the second-direction wire 340 so that the hook may be stably fixed to the occluder 300 through the first positioning portion 111 and the second positioning portion 112. In addition, the first positioning portion 111 and the second positioning portion 112 may be fixed to the same direction wire at the same time under the premise of different directions of force, for example, being fixed to the first-direction wire 330 at the same time or being fixed to the second-direction wire 340 at the same time, thereby stably fixing the hook to the occluder 300 under the complementary condition. The hook portion 120 is formed by bending the positioning body 110. The hook is fixed to the first-direction wire 330/the second-direction wire 340 axially. Thus, the positioning body 110 is fixed to the occluder 300 with the axis M parallel to the first-direction wire 330 or the second-direction wire 340. When the included angle α between the projection of the hook portion 120 on the positioning body 110 and the axis M of the positioning body 110 is in a range of greater than 0 degrees and less than 90 degrees, a stable fixed angle between the hook portion 120 and the left atrial appendage 400 may be ensured when the hook is fixed to the occluder 300 so as to limit the disengagement of the occluder 300 from the left atrial appendage 400.

Embodiment 1, a first aspect of the present application will first illustrate a hook connected to a fixing disc by perforation.

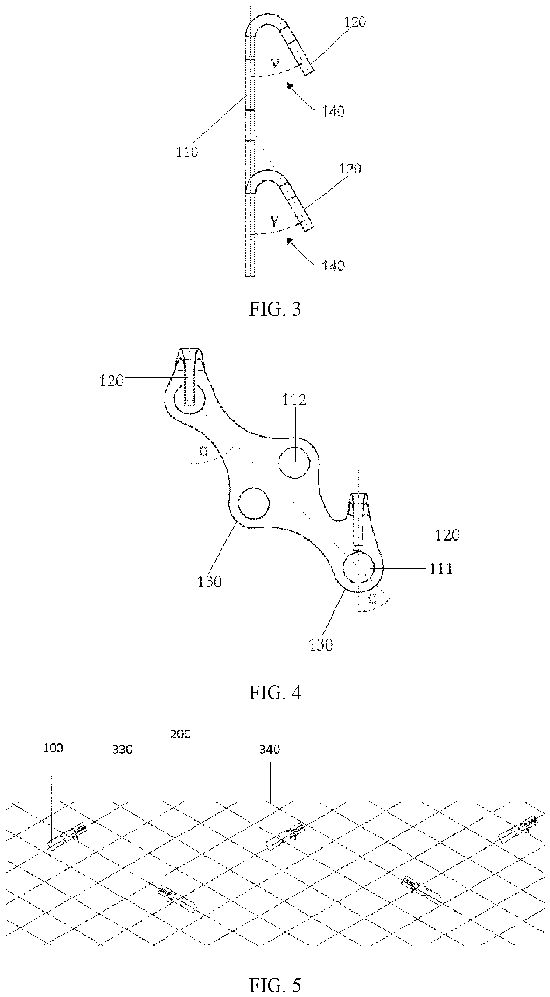

Referring to FIGS. 2 and 3, in this embodiment, the positioning body 110 is substantially strip-shaped, two bulging portions 130, which are on the axis M of the positioning body, are formed at the two end portions of the positioning body 110, respectively, and a middle part of the two end portions also has two bulging portions 130 protruding to two sides. The first positioning portion 111 includes first positioning holes arranged on the two bulging portions 130 at the end portions, and the second positioning portion 112 includes second positioning holes arranged on the two bulging portions 130 at the middle. A connecting line between the second positioning holes and a connecting line between the first positioning holes have an included angle β, and a value of the included angle β is equal to a value of an included angle between the first-direction wire and the second-direction wire. One of the first-direction wire 330 and the second-direction wire 340 is threaded through the first positioning holes, and the other of the first-direction wire 330 and the second-direction wire 340 is threaded through the second positioning holes. Illustratively, when the first-direction wire 330 is threaded through the first positioning holes, the second-direction wire 340 is threaded through the second positioning holes. In actual use, referring to FIG. 1, a node of the first-direction wire 330 and the second-direction wire 340 may also be threaded through the first positioning holes, i.e., both the first-direction wire 330 and the second-direction wire 340 may be threaded through the first positioning holes at the same time so as to more stably fix the hook to the occluder 300. In this embodiment, the bulging portion 130 may expand the area of the positioning body 110 where the bulging portion 130 is located so as to arrange the first positioning hole or the second positioning hole and increase the strength of the positioning body 110 at the same time. The bulging portion 130 does not have to be provided, and the first positioning hole or the second positioning hole may be directly arranged at the corresponding position of the positioning body 110. In addition, on the basis that the first-direction wire 330 and the second-direction wire 340 may have a small range of deformation, the included angle between the connecting line of the second positioning holes and the connecting line of the first positioning holes is approximately equal to the included angle between the first-direction wire and the second-direction wire.

The hook portion 120 is formed by bending the positioning body 110, and specifically, is formed by extending and bending the positioning body 110 beside each first positioning hole (referring to FIGS. 1-2 and 4, where the orientations of the components are consistent, for example, left or right). The bending angles γ formed between each the hook portions 120 and the positioning body 110 are the same. In this embodiment, there are a total of two first positioning holes, and accordingly, two hook portions 120 are provided. Referring to FIGS. 2 and 3, the bending angles γ formed between the two hook portions 120 and the positioning body 110 are the same, i.e., in terms of the bending direction, as viewed from the front of the hook, the projections of the two hook portions 120 on the positioning body 110 and the axis M of the positioning body 110 both form included angles α, and the included angle α is in a range of greater than 0 degrees and less than 90 degrees, as shown in FIG. 2. In terms of the bending angle, as viewed from the side surface of the hook, as shown in FIG. 3, the bending angles of the two hook portions 120 with respect to the positioning body 110 are both γ, and setting the bending angles and the bending directions to be the same may ensure that the hook portions 120 hook in the same direction to the left atrial appendage 400 during use, and the resultant force is great, thereby increasing the effective occluding of the left atrial appendage by the occluder 300.

In this embodiment, with continuous reference to FIGS. 1-2 and 4, when the projections of the hook portions 120 on the positioning body 110 and the axis of the positioning body 110 form included angles α, and the included angle α is in a range of greater than 0 degrees and less than 90 degrees, there may be two bending directions of the hook portion 120, i.e., the projection of the hook portion 120 on the positioning body 110 is located on a first side or a second side of the axis of the positioning body 110. The first side is an opposite side of the second side. The hook shown in FIG. 2 is a first hook 100, and a projection of the hook portion 120 of the first hook on the positioning body 110 is located on a first side (left side shown in FIG. 2) of the axis of the positioning body 110. The first hook 100 is configured to limit the axial direction to the first-direction wire 330 through the first positioning portion 111. With specific reference to FIGS. 1 and 4, the hook mirror-symmetrical to the first hook 100 shown in FIG. 2 is a second hook 200, and a projection of the hook portion 120 of the second hook 200 on the positioning body 110 is located on a second side (right side shown in FIG. 4) of the axis of the positioning body 110. The second hook 200 is configured to limit the axial direction to the second-direction wire 340 through the first positioning portion 111. The axial direction of the hook is limited to the first-direction wire 330 or the second-direction wire 340. It can be understood that the axial direction of the hook is parallel to the first-direction wire 330 or the second-direction wire 340. That is, the axis M of the first hook 100 is limited to the first-direction wire 330, and the axis M of the second hook is limited to the second-direction wire 340.

Embodiment 2, a second aspect of the present application will illustrate the hook fixed to the fixing disc 310 by press-fitting.

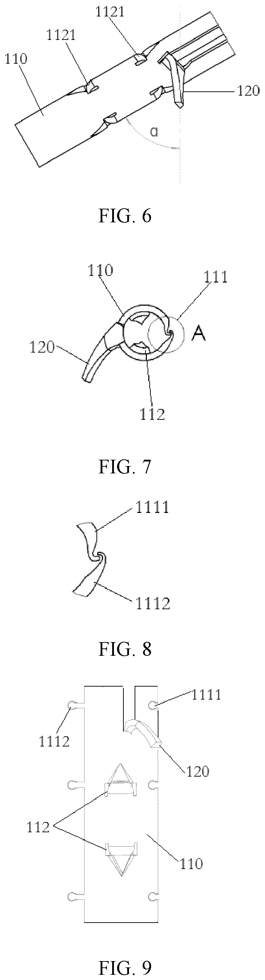

Referring to FIG. 5-11, in this embodiment, a length-width ratio of the positioning body 110 is greater than 1: 1. Two long edges of the positioning body 110 are set as a first edge and a second edge which are opposite to each other, respectively. The first positioning portion 111 includes first parts 1111 and second parts 1112 which are provided on the first edge and the second edge, respectively, and the first parts 1111 and the second parts 1112 are detachably connected. The first edge and the second edge are bendable towards each other to fix the positioning body 110 to the first-direction wire 330/the second-direction wire 340 by buckling the first parts 1111 and the second parts 1112. Specifically, when the hook is normally expanded as shown in FIG. 9, the positioning body 110 may be bent to wrap the first-direction wire 330 or the second-direction wire 340. When the positioning body 110 is bent, the first parts 1111 is buckled with the second parts 1112). That is, in this embodiment, the shape of the positioning body 110 in the use state may be considered to have a cylindrical shape which may be expanded and closed in the axial direction. The manner of expanding and closing is mainly achieved by the cooperation of the first parts 1111 and the second parts 1112. Referring to FIGS. 7 and 8, the first parts 1111 and the second parts 1112 may be clasps buckled with each other. Referring to FIG. 9, the first parts 1111 may be several catching grooves arranged on the first edge in the axial direction, and the second parts 1112 may be connecting convex portions which correspond to and are adapted to the catching grooves one by one.

Referring to FIG. 6, the second positioning portion 112 includes at least one pair of oppositely provided barbs 1121, and the two barbs 1121 are provided along the axial direction of the positioning body. The second positioning portion 112 may include a plurality of pairs of barbs 1121. For example, the second positioning portion 112 includes two pairs of barbs 1121, and the two pairs of barbs 1121 are provided flush in the axial direction of the positioning body 110.

In this embodiment, referring to FIG. 6 for the structure of the hook portion 120, it is formed by bending the positioning body 110 after cutting. Specifically, the hook portion 120 is a strip which is cut from an end portion of the positioning body 110 in the axial direction and connected to the positioning body 110 at the root. The barb 1121 is extended rearward to be inserted to the first-direction wire 330 or the second-direction wire 340, and the strip is bent forward obliquely to form the hook portion 120.

In this embodiment, the projections of the hook portions 120 on the positioning body 110 are located on different sides of the axis of the positioning body 110 so that two types of hooks-a first hook 100 and a second hook 200 may also be formed. The projection of the hook portion 120 of the first hook on the positioning body 110 is located on a first side of the axis of the positioning body 110, and referring to FIG. 5 or FIG. 6, the first side is a right side of the axis of the positioning body 110. At this time, the root of the hook portion 120 is located on a left side of the axis of the positioning body 110. The projection of the hook portion 120 of the second hook 120 on the positioning body 110 is located on a second side of the axis of the positioning body 110, and referring to FIG. 5, the second side is the left side of the axis of the positioning body 110. At this time, the root of the hook portion 120 is located on the right side of the axis of the positioning body 110.

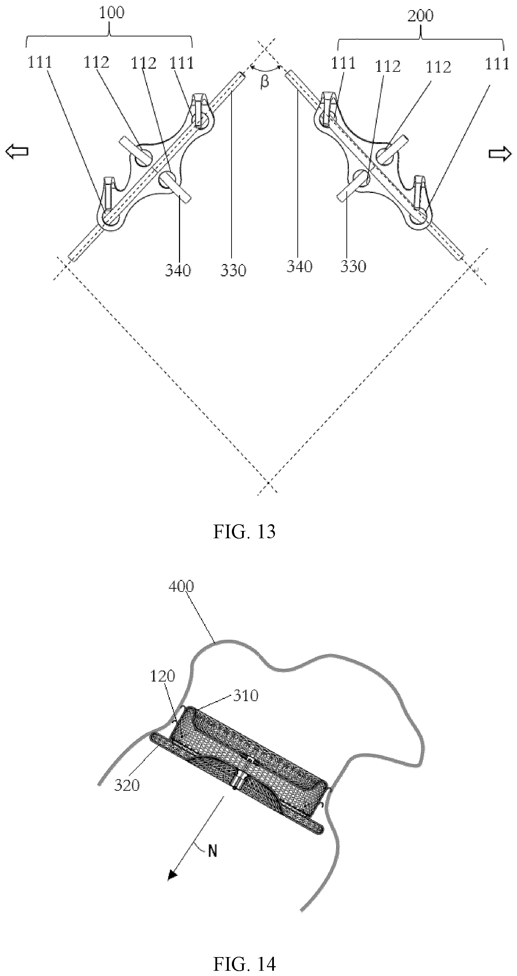

When the first hook 100 is mounted on the first-direction wire 330 and the second hook 200 is mounted on the second-direction wire, the hook portion 120 of the first hook 100 and the hook portion 120 of the second hook 200 face substantially the same direction, as specifically shown in FIG. 13.

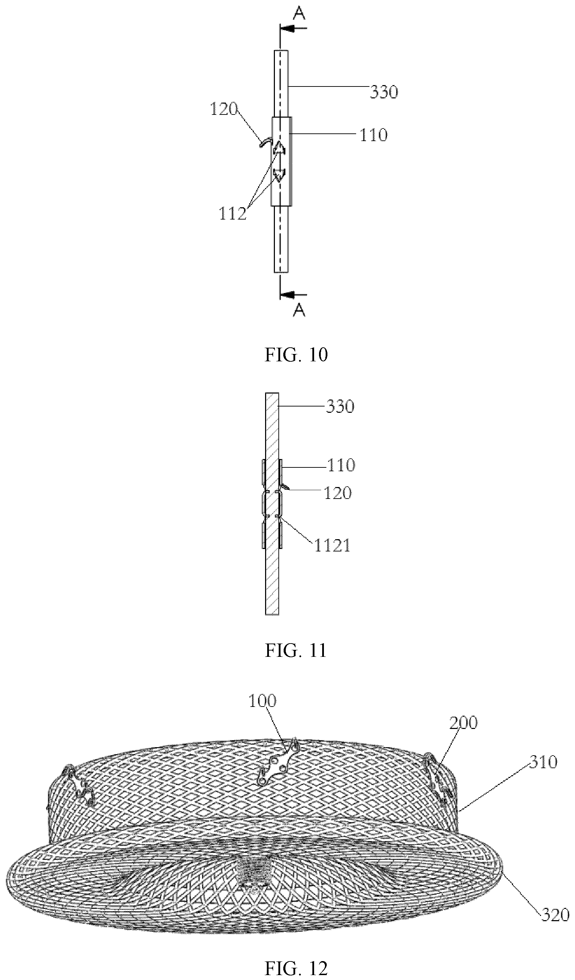

During use, referring to FIGS. 10 and 11, the first edge and the second edge of the positioning body 110 are bent towards each other, and the positioning body 110 sheathes the first-direction wire 330 or the second-direction wire 340. At this time, the barbs 1121 are bent towards the fixing disc 310, and the hook portion 120 is bent away from the fixing disc 310. That is, the bending direction of the barbs 1121 is different from that of the hook portion 120. At this time, the barbs 1121 of the second positioning portion 112 are pressed into the corresponding first-direction wire 330 or second-direction wire 340 by press-fitting. Finally, the positioning body is buckled in the axial direction on the first-direction wire 330 or the second-direction wire 340 through the first parts 1111 and the second parts 1112 of the first positioning portion 111 so that the hook is fixed to the fixing disc 310.

Embodiment 3, a third aspect of the present application will illustrate an occluder 300.

Referring to FIG. 12, the occluder 300 is formed by interleaving a first-direction wire 330 and a second-direction wire 340, including a fixing disc 310 and an occluding disc 320. The fixing disc 310 is provided with several hooks and used for being fixed in a left atrial appendage 400 through the hooks. The occluding disc 320 has a diameter greater than the fixing disc 310 and is configured to occlude the left atrial appendage 400.

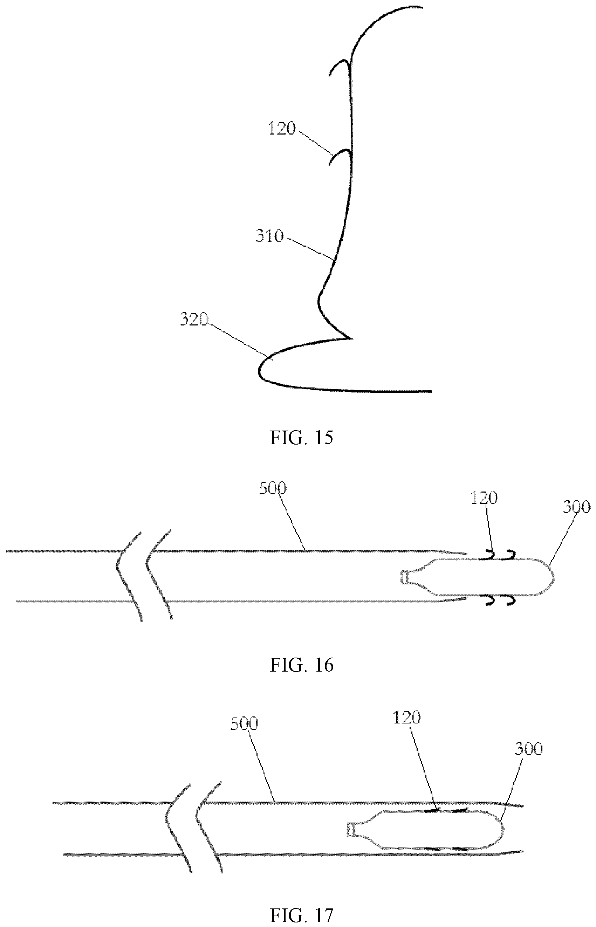

The hooks of this embodiment are the hooks described in the first aspect, including a first hook 100 and a second hook 200. The first hook 100 and the second hook 200 both have two hook portions 120 provided above and below. The included angles α between the projections of the hook portions 120 on the positioning body 110 and the axis of the positioning body 110 are equal, and the bending angles γ between the hook portions 120 and the positioning body 110 are also equal. Referring to FIG. 13, during the process of weaving the fixing disc 300, the first-direction wire 330 passes through two first positioning holes of the first hook 100, and the second-direction wire 340 passes through two second positioning holes of the first hook 100. Since the first-direction wire 330 and the second-direction wire 340 form a cross structure, the first hook 100 is fixed, and the second hook 200 is fixed in the same manner. When second-direction wire 340 is threaded through the first positioning holes of the second hook 200, the first-direction wire 330 is threaded through the second positioning holes. In use, referring to FIG. 14, when the occluder 300 is implanted, the fixing disc 310 is compressed by the differently shaped left atrial appendage 400, causing the deformation of the fixing disc 310. Referring to FIG. 15, since the hook is relatively fixed to the fixing disc 310, the deformation of the fixing disc 310 will drive the hook to play a fixing role at different positions within the left atrial appendage 400, thereby ensuring the stability of the occlusion.

In this way, the fixing manner of the structure of the hook completely eliminates other auxiliary components for fixation, thereby improving the stability and firmness of the hook, and meanwhile greatly reducing the volume of the non-degradable material. In addition, the first hooks 100 and the second hooks 200 are spaced apart on the fixing disc 310 of the occluder 300, and a plurality of hooks are arranged in a wavy line on the fixing disc 300. The adjacent first hook 100 and second hook 200 form two adjacent edges of an approximate diamond shape, thus increasing the supporting strength of the occluding disc 320 and making the occluding effect more stable.

FIG. 16 is a schematic diagram of an occluder 300 being released through a delivery sheath 500, and FIG. 17 is a schematic diagram of an occluder 300 being retrieved through a delivery sheath 500. After the occluder 300 is released from the delivery sheath 500, the hook portion 120 will immediately return to the shaped J-shaped structure state due to its memory property. When the occluder 300 is retrieved into the delivery sheath 500, the hook portion 120 is linearly expanded within the delivery sheath 500 after being subjected to a certain force.

Embodiment 4, a fourth aspect of the present application will illustrate another occluder 300.

The occluder 300 in this embodiment differs from the occluder 300 provided in the fourth aspect in that the hook adopts the hook described in the second aspect. That is, the positioning body 110 of the hook has a first edge and a second edge which are opposite to each other. The first positioning portion 111 includes first parts 1111 and second parts 1112 which are provided on the first edge and the second edge, respectively, and the first parts 1111 and the second parts 1112 are detachably connected. The second positioning portion 112 includes at least one pair of oppositely provided barbs 1121, and the two barbs 1121 are provided along the axial direction of the positioning body. The positioning body 110 wraps around the designated first-direction wire 330/the designated second-direction wire 340 after bending the first edge and the second edge towards each other, the first parts 1111 and the second parts 1112 of the first positioning portion 111 are buckled with each other so that the positioning body 110 sheathes the designated first-direction wire 330/the designated second-direction wire 340, and the barbs 1121 puncture the designated first-direction wire 330/the designated second-direction wire 340 for fixing under an external pressure. In order to improve the stability of the hook, the first-direction wire 330 and the second-direction wire 340 intersect to form a node, and two barbs 1121 may be fixed at two sides of the node on the designated first-direction wire 330/the designated second-direction wire 349, respectively.

Similarly, like the occluder 300 described in the third aspect, the hooks in this embodiment also include a first hook 100 and a second hook 200. The hook-shaped directions of the hook portions 120 in the first hook 100 and the second hook 200 are opposite to each other, and the distribution of the first hook 100 and the second hook 200 on the framework of the occluder 100 is staggered up and down and spaced apart. This ensures a uniform and efficient distribution of the hooks on the fixing disc 310.

It should be noted that for the occluder in the first aspect, the second aspect, the third aspect, or the fourth aspect, when the first hook 100 is mounted on the first-direction wire 330 of the fixing disc 310 and the second hook 200 is mounted on the second-direction wire 340 of the fixing disc 310, an opening between the hook portion 120 and the positioning body 110 of the first hook 100 faces the occluding disc 320, and an opening between the hook portion 120 and the positioning body of the second hook 200 faces the occluding disc 320 so that the fixing disc 310 is mounted on the left atrial appendage 400 through the hook portions 120. Specifically, the fixing disc 310 is positioned further inward of the left atrial appendage 400 than the occluding disc 320, and if the occluder 300 is disengaged from the left atrial appendage 400, the fixing disc 310 needs to move away from the interior of the left atrial appendage 400, i.e., to the occluding disc 320 side. The opening between the hook portion 120 and the positioning body 110 is oriented in the same direction as the moving direction N of the fixing disc 310. At this time, when the occluder 300 is placed in the left atrial appendage 400, the hook portion 120 may be inserted into the left atrial appendage 400, thereby limiting disengagement of the occluder 300. More specifically, as shown in FIGS. 2 and 3, one end of the hook portion 120 is connected to the positioning body 110, and the other end of the hook portion 120 forms an opening with the positioning body 110 and extends in the same direction as that of the fixing disc 310 towards the occluding disc 320, i.e., the moving direction N.

It is to be understood that the same or similar parts in the above-mentioned embodiments may be referred to each other. What is not described in detail in some embodiments may be referred to the same or similar content in other embodiments. It should be noted that, in the description of the present application, terms are defined in consideration of functions in the present invention and may be different according to the intention or convention of a user or an operator. Therefore, the definitions of such terms should be defined based on the whole content of this specification.

For example, orientation or positional relationships indicated by the terms "center", "upper", "lower", "left", "right", "vertical", "horizontal", "inner", "outer", and the like are based on the orientation or positional relationships shown in the drawings, and are intended only to facilitate and simplify the description of the present invention, and are not intended to indicate or imply that the apparatus or element referred to must have a particular orientation, constructed and operated in a particular orientation, and therefore are not to be construed as limitations of the present invention. In addition, the terms "first", "second", and "third" are used for descriptive purposes only and are not to be construed as indicating or implying relative importance.

In the description of the specification, unless otherwise explicitly stated or limited, the terms "mounted", "communicated", and "connected" are to be construed broadly, such as fixedly connected, detachably connected, integrally connected, mechanically connected, electrically connected, directly connected, indirectly connected through an intermediate medium, and communicated between two elements. The specific meanings of the above-mentioned terms in the present invention may be understood by a person skilled in the art according to specific circumstances.

The description with reference to the terms "one embodiment", "some embodiments", "example", "specific example", or "some examples", etc. means that specific features, structures, materials, or characteristics described in connection with the embodiment or example is included in at least one embodiment or example of the present application. In this specification, schematic representations of the above-mentioned terms do not necessarily refer to the same embodiment or example. Furthermore, the specific features, structures, materials, or characteristics described may be combined in any suitable manner in any one or more embodiments or examples.

While embodiments of the present application have been shown and described above, it is to be understood that the above-mentioned embodiments are illustrative and are not to be construed as limiting the present application. Changes, modifications, substitutions, and variations of the above-mentioned embodiments may be made by a person skilled in the art within the scope of the present application.

## Claims

1. A hook, **characterized by** being used for being fixed to an occluder,
wherein the occluder is formed by interweaving a first-direction wire and a second-direction wire, and the hook comprises a positioning body and hook portions, wherein
the positioning body is provided with a first positioning portion and a second positioning portion; the first positioning portion is configured to limit the hook to one of the first-direction wire and the second-direction wire, and the second positioning portion is configured to limit the hook to the other of the first-direction wire and the second-direction wire;
or the first positioning portion and the second positioning portion are configured to limit, in different directions of force, the hook in an axial direction to the first-direction wire or the second-direction wire;
the hook portion is formed by bending the positioning body; an included angle α is formed between a projection of the hook portion on the positioning body and an axis of the positioning body, and the included angle α is in a range of greater than 0 degrees and less than 90 degrees.

2. The hook according to claim 1, **characterized in that**, the first positioning portion comprises at least two first positioning holes arranged on the positioning body, and the two first positioning holes are distributed along the axial direction; the second positioning portion comprises at least two second positioning holes; one of the first-direction wire and the second-direction wire is threaded through the first positioning holes, and the other of the first-direction wire and the second-direction wire is threaded through the second positioning holes.

3. The hook according to claim 2, **characterized in that**, a connecting line between the second positioning holes and a connecting line between the first positioning holes have an included angle β, and a value of the included angle β is equal to a value of an included angle between the first-direction wire and the second-direction wire.

4. The hook according to claim 2 or 3, **characterized in that**, there are two first positioning holes, and the two first positioning holes are arranged at two ends of the positioning body, respectively.

5. The hook according to any one of claims 2 to 4, **characterized in that**, bulging portions are formed on the positioning body corresponding to the first positioning holes and the second positioning holes.

6. The hook according to any one of claims 2 to 5, **characterized in that**, one hook portion is provided beside each first positioning hole; the hook portions are formed by extending and bending the positioning body, and bending angles γ formed between the hook portions and the positioning body are the same.

7. The hook according to claim 1, **characterized in that**, the positioning body has a first edge and a second edge which are opposite to each other; the first positioning portion comprises first parts and second parts which are provided on the first edge and the second edge, respectively, and the first parts and the second parts are detachably connected; the first edge and the second edge are bendable towards each other to fix the positioning body to the first-direction wire or the second-direction wire by buckling the first parts and the second parts.

8. The hook according to claim 7, **characterized in that**, the first parts and the second parts are clasps buckled with each other.

9. The hook according to claim 7 or 8, **characterized in that**, the first parts are several catching grooves arranged on the first edge in the axial direction, and the second parts are connecting convex portions which correspond to and are adapted to the catching grooves one by one.

10. The hook according to any one of claims 7 to 9, **characterized in that**, the second positioning portion comprises at least one pair of oppositely provided barbs; and the two pair of barbs are provided along the axial direction of the positioning body.

11. The hook according to claim 10, **characterized in that**, the second positioning portion comprises two pairs of barbs, and the two pairs of barbs are provided flush in the axial direction.

12. The hook according to any one of claims 7 to 11, **characterized in that**, the hook portion is formed by bending the positioning body after cutting.

13. The hook according to claim 12, **characterized in that**, the hook portion comprises a strip which is cut from an edge of the positioning body and connected to the positioning body at the root; the strip is bent away from a bending direction of the barb.

14. The hook according to any one of claims 1 to 13, **characterized in that**, the projection of the hook portion on the positioning body is located on a first side or a second side of the axis of the positioning body, wherein the first side is an opposite side of the second side.

15. The hook according to claim 14, **characterized in that**, when the projection of the hook portion on the positioning body is located on the first side of the axis of the positioning body, the first positioning portion is configured to limit the hook in the axial direction to the first-direction wire; when the projection of the hook portion on the positioning body is located on the second side of the axis of the positioning body, the first positioning portion is configured to limit the hook in the axial direction to the second-direction wire.

16. An occluder, **characterized by** being formed by interweaving a first-direction wire and a second-direction wire, comprising:
a fixing disc, provided with several hooks according to any one of claims 1 to 15 and used for being fixed in a left atrial appendage through the hooks; and
an occluding disc having a diameter greater than the fixing disc, configured to occlude the left atrial appendage.

17. The occluder according to claim 16, **characterized in that**, the hooks are provided on the first-direction wire and the second-direction wire of the fixing disc, and hanging directions of the hooks are the same.

18. The occluder according to claim 17, **characterized in that**, the hooks comprise first hooks and second hooks; the first hook is limited to the first-direction wire through the first positioning portion, and the second hook is limited to the second-direction wire through the first positioning portion, wherein the hook portions of the first hook and the hook portions of the second hook are oriented in the same direction.

19. The occluder according to claim 16, **characterized in that**, the positioning body has a first edge and a second edge which are opposite to each other; the first positioning portion comprises first parts and second parts which are provided on the first edge and the second edge, respectively, and the first parts and the second parts are detachably connected;
the second positioning portion comprises at least one pair of oppositely provided barbs; and the two barbs are provided along the axial direction of the positioning body;
the positioning body wraps around the first-direction wire or the second-direction wire after bending the first edge and the second edge towards each other, the first parts and the second parts of the positioning body are buckled with each other so that the positioning body sheathes the first-direction wire or the second-direction wire, and the barbs puncture the first-direction wire or the second-direction wire for fixing under an external pressure;
the first-direction wire and the second-direction wire intersect to form a node, and the two barbs are fixed at two sides of the node on the first-direction wire or the second-direction wire, respectively.

20. The occluder according to claim 19, **characterized in that**, the hooks comprise first hooks and second hooks; the positioning body of the first hook wraps around the first-direction wire after bending the first edge and the second edge towards each other, and the positioning body of the second hook wraps around the second-direction wire after bending the first edge and the second edge towards each other, wherein the hook portion of the first hook and the hook portion of the second hook are oriented in the same direction.

21. The occluder according to any one of claims 16 to 20, **characterized in that**, when the first hook is mounted on the first-direction wire of the fixing disc and the second hook is mounted on the second-direction wire of the fixing disc, an opening between the hook portion and the positioning body of the first hook faces the occluding disc, and an opening between the hook portion and the positioning body of the second hook faces the occluding disc.

22. The occluder according to any one of claims 16 to 21, **characterized in that**, the hooks are arranged in a wavy line along a circumferential direction of the fixing disc.
